# FASCICULE DE BREVET EUROPEEN

(11) **EP 0 938 473 B1**
(45) Date de publication et mention de la délivrance du brevet: **11.12.2002**
(21) Numéro de dépôt: 97912244.7
(22) Date de dépôt: 23.10.1997
(51) Int. Cl.: C07D 201/08

(54) **PROCEDE DE TRAITEMENT DE LACTAMES**
BEHANDLUNGSVERFAHREN VON LAKTAMEN
METHOD FOR TREATING LACTAMS

(30) Priorité: 24.10.1996 FR 9613203
(43) Date de publication de la demande: 01.09.1999
(73) Titulaire: RHODIA POLYAMIDE INTERMEDIATES, 69190 Saint-Fons (FR)
(72) Inventeur: CHIARELLI, Henri, F-69360 Communay (FR); LECONTE, Philippe, F-69330 Meyzieu (FR)
(74) Mandataire: Esson, Jean-Pierre
(86) Numéro de dépôt international: FR9701903
(87) Numéro de publication internationale: WO98017641

(56) Documents cités:
- EP-A- 0 659 741
- WO-A-96/22974

## Description

La présente invention a trait au traitement de lactames directement issus de leur procédé de synthèse, en évitant le plus possible la formation d'oligomères.

Les lactames, et plus particulièrement le caprolactame qui est la matière de base du polyamide 6, sont généralement distillés pour être séparés des composés à partir desquels ils ont été préparés et des sous-produits également formés lors de leur synthèse.

Le caprolactame est préparé industriellement de manière classique par une réaction de réarrangement de Beckmann de l'oxime de la cyclohexanone avec de l'acide sulfurique ou de l'oléum, suivie de la neutralisation du milieu par l'ammoniac, puis de la séparation et de la purification du caprolactame.

Les lactames peuvent également être issus de l'hydrolyse cyclisante des aminonitriles. Ils doivent alors être séparés de l'ammoniac formé, de l'eau non transformée, du solvant éventuellement utilisé, des composés organiques légers (c'est-à-dire ayant un point d'ébullition inférieur à celui du lactame), de l'aminonitrile éventuellement non transformé, ainsi que des composés organiques plus lourds (de point d'ébullition plus élevé que celui du lactame).

La purification du lactame, principalement par distillation, et plus particulièrement sa séparation de l'eau, n'est pas toujours effectuée immédiatement à la sortie du réacteur d'hydrolyse cyclisante.

Il a été observé qu'il se forme des oligomères du lactame, lorsque le flux réactionnel sortant du réacteur d'hydrolyse est maintenu pendant un laps de temps relativement long à une température supérieure à 50°C environ. En effet le maintien du mélange réactionnel en phase condensée liquide entraîne la formation d'oligomères.

De même, il a été constaté que si la durée moyenne de séjour du lactame dans l'appareil de distillation excède environ une heure à une température supérieure à 100°C, il se forme également des oligomères.

En outre, lorsque la distillation du lactame n'est pas effectuée immédiatement après sa préparation, ledit lactame est parfois stocké pendant une durée pouvant être relativement longue, c'est-à-dire à titre indicatif égale à plusieurs heures.

Il a été observé que le stockage pendant une durée de quelques heures du lactame brut, notamment en solution, à une température égale ou supérieure à 50°C conduit à la formation d'oligomères dudit lactame.

Selon l'appareillage utilisé, la présence de ces oligpmères formés lors des différentes phases du traitement du flux issu de la réaction d'hydrolyse cyclisante, est susceptible de provoquer des bouchages, notamment dans les conduites entre le réacteur et la colonne de distillation ou le stockeur. En outre, la formation d'oligomères réduit le rendement global en lactame et crée des problèmes supplémentaires de purification et de recyclage.

La présente invention remédie à ces inconvénients en limitant le plus possible la formation des oligomères du lactame.

Elle consiste plus précisément en un procédé de traitement d'un lactame à partir du flux réactionnel issu d'une hydrolyse cyclisante en phase vapeur d'un aminonitrile, caractérisé en ce que le flux réactionnel sortant du réacteur d'hydrolyse est refroidi en un laps de temps inférieur ou égal à 10 minutes
- à une température inférieure ou égale à 150°C avant d'être distillée,
- ou à une température inférieure ou égale à 50°C avant d'être stocké pendant au moins une heure, avant d'être fractionnée.

Le refroidissement peut être effectué dans les procédés industriels par un échangeur thermique à circulation d'eau, d'air ou le cas échéant de vapeur ou encore de fluide caloporteur.

En pratique, une variante d'exécution du refroidissement consistera à récupérer les calories du mélange réactionnel issu de la réaction d'hydrolyse.

Ces calories peuvent être utilisées directement pour préchauffer un fluide caloporteur pouvant servir ensuite à préchauffer les réactifs de l'hydrolyse cyclisante, c'est-à-dire essentiellement l'aminonitrile et l'eau.

Elles peuvent aussi servir à préchauffer la vapeur d'eau utilisée pour chauffer le réacteur d'hydrolyse.

Elles peuvent également servir à générer de la vapeur d'eau qui sera utilisée dans une phase ou l'autre du procédé de préparation des lactames.

Cette récupération des calories du mélange issu de la réaction d'hydrolyse cyclisante peut être complétée par un refroidissement à l'eau ou à l'air, lorsque la température dudit mélange atteint une valeur inférieure à 150°C ou de préférence inférieure à 100°C.

Dans le présent texte, le terme refroidissement englobe les différents modes de réalisation indiqués précédemment ou équivalents.

Lorsque le flux réactionnel est distillé sans délai, c'est-à-dire sans stockage, il est préférable pour l'économie du procédé de refroidir en un laps de temps le plus court possible, de préférence inférieur ou égal à 10 minutes, ledit flux à une température de 50°C à 150°C et de préférence de 70°C à 120°C. Il est également recommandé de limiter la durée d'alimentation de la colonne de distillation, c'est-à-dire le temps qui s'écoule entre la sortie du réacteur d'hydrolyse du flux réactionnel et la distillation proprement dite, à une heure au maximum et de préférence à 30 minutes. La distillation elle-même est conduite de manière à ce que le temps moyen de séjour du lactame dans l'appareil de distillation soit inférieur ou égal à 1 heure.

Lorque le flux réactionnel n'est pas traité immédiatement et est stocké pendant une durée supérieure à 1 heure, le refroidissement est effectué en un laps de temps inférieur ou égal à 10 minutes et de préférence à une température inférieure ou égale à 50°C.

Le lactame mis en oeuvre dans le présent procédé est plus particulièrement choisi parmi ceux qui sont obtenus par hydrolyse cyclisante en phase vapeur, d'un aminonitrile aliphatique de formule générale (I) :

N≡C―R―NH₂ (I)

dans laquelle R représente un radical alkylène linéaire ou ramifié ayant de 3 à 12 atomes de carbone.

Parmi les lactames, on peut plus particulièrement citer ceux qui servent de matière première pour la préparation des polyamides 4, 5, 6 et 11 et qui sont obtenus à partir des aminonitriles de formule (I), dans laquelle le symbole R représente un radical alkylène linéaire ayant 3, 4, 5 ou 10 atomes de carbone.

Comme indiqué précédemment, le caprolactame dont la polymérisation fournit le polyamide 6, qui est préparé à partir de l'amino-6 capronitrile (ou epsilon-capronitrile), est le lactame préférentiellement mis en oeuvre dans le procédé de l'invention.

A titre d'illustration non limitative du procédé de préparation de lactames, par hydrolyse cyclisante en phase vapeur d'aminonitriles de formule (I), on peut se référer par exemple aux brevets EP-A-0 659 741 ou WO-A-96/22974.

Le lactame à purifier est généralement sous forme d'une solution aqueuse ou alcoolique. L'hydrolyse cyclisante étant effectuée en phase vapeur, ledit lactame obtenu est le plus souvent en solution aqueuse. La concentration en lactame d'une telle solution est en général de 20 % à 80 % en poids par poids. L'aminonitrile représente habituellement jusqu'à 15 % du poids du lactame et le plus fréquemment de 0 % à 10 % de ce poids.

Le procédé de l'invention permet de manière générale de limiter la formation d'oligomères à une teneur telle que le phénomène soit pratiquement sans conséquence néfaste. Une telle teneur est généralement inférieure ou égale à 2 % en poids d'oligomères par poids de lactame et de préférence inférieure ou égale à 1 % en poids par poids.

De préférence, dans le procédé de l'invention, le lactame est conservé à une température inférieure ou égale à 40°C avant sa distillation si la durée de stockage est supérieure ou égale à 1 heure et/ou en ce que la distillation est conduite de manière à ce que le temps moyen de séjour du lactame dans l'appareil de distillation soit inférieur ou égal à 30 minutes.

Les exemples qui suivent illustrent l'invention.

### Exemple 1

On réalise l'hydrolyse cyclisante de l'amino-6 capronitrile par passage de 91g/h de ce composé et de 85 g/h d'eau à 300°C, sur 53 ml (22,7 g) d'alumine.

En sortie de réacteur, les gaz sont refroidis rapidement (moins de 5 minutes) à température ambiante (environ 20°C).

On obtient une solution aqueuse ammoniacale limpide contenant (dosage chromatographique) 47,5 % en poids de caprolactame et 2,9 % en poids d'aminocapronitrile. Cela correspond à un taux de transformation (TT) de l'aminocapronitrile de 94 % et à un rendement en caprolactame par rapport à l'aminocapronitrile chargé (RR) de 93 % (soit une sélectivité en caprolactame par rapport à l'aminocapronitrile transformé ou RT de 99 %)

On stocke cette solution pendant 3 mois à une température de 20°C environ, puis on la dose à nouveau par chromatographie. On n'observe pas de présence de précipité. On trouve alors 48 % en poids de caprolactame et 2,7 % en poids d'aminocapronitrile, ce qui, compte tenu de la précision des dosages, signifie que ces solutions brutes de caprolactame sont stables dans ces conditions.

### Essai comparatif 1

On maintient une solution aqueuse contenant 50 % en poids d'un mélange équimoléculaire de caprolactame et d'ammoniac à 150°C pendant 6 heures. Par dosage on constate que 10 % du caprolactame ont été transformés, alors qu'un précipité de couleur brun-rouge s'est formé.

### Exemple 2

On réalise l'hydrolyse cyclisante de l'amino-6 capronitrile par passage de 100 g/h de ce composé et de 64 g/h d'eau à 300°C, sur 200 ml (135 g) d'alumine.

En sortie de réacteur, les gaz sont refroidis rapidement (moins de 5 minutes) à température ambiante (environ 20°C).

On obtient une solution aqueuse ammoniacale limpide contenant (dosage chromatographique) du caprotactame et de l'aminocapronitrile en quantités correspondant à un taux de transformation de l'aminocapronitrile de 95,5 % et à un rendement en caprolactame par rapport à l'aminocapronitrile chargé de 95,5 % (soit une sélectivité en caprolactame par rapport à l'aminocapronitrite transformé de 100 %)

### Essai comparatif 2

On réalise l'hydrolyse cyclisante de l'amino-6 capronitrile par passage de 100 g/h de ce composé et de 64 g/h d'eau à 300°C, sur 200 ml (135 g) de l'alumine utilisée dans l'exemple 2.

En sortie de réacteur, le flux réactionnel est refroidi et maintenu à environ 150°C pendant deux heures (au total), avant d'être refroidi en 10 secondes à température ambiante (environ 20°C). Pendant cette période de maintien à 150°C, le flux comporte alors une phase gazeuse et une phase condensée liquide.

On obtient une solution aqueuse ammoniacale contenant (dosage chromatographique) du caprolactame et de l'aminocapronitrile en quantités correspondant à un taux de transformation de l'aminocapronitrile de 98,5 % et à un rendement en caprolactame par rapport à l'aminocapronitrile chargé de 94 % (soit une sélectivité en caprolactame par rapport à l'aminocapronitrile transformé de seulement 95 %). Cette solution contient un précipité d'oligomères de caprolactame.

## Revendications

1. Procédé de séparation d'un lactame à partir du flux réactionnel issu d'une hydrolyse cyclisante en phase vapeur d'un aminonitrile, **caractérisé en ce que** le flux réactionnel sortant du réacteur d'hydrolyse est refroidi en un laps de temps inférieur ou égal 10 minutes :
- à une température de 50°C à 150°C avant d'être distillé,
- ou à une température inférieure ou égale à 50°C avant d'être stocké, pendant au moins une heure, avant d'être fractionnée.

2. Procédé selon la revendication 1, **caractérisé en ce que** le flux réactionnel est refroidi à une température de 70°C à 120°C avant d'être distillé.

3. Procédé selon l'une des revendications 1 ou 2, **caractérisé en ce que** l'on limite la durée d'alimentation de la colonne de distillation, c'est-à-dire le temps qui s'écoule entre la sortie du réacteur d'hydrolyse du flux réactionnel et la distillation proprement dite, à une heure au maximum et de préférence à 30 minutes.

4. Procédé selon l'une des revendications 1 à 3, **caractérisé en ce que** la distillation est conduite de manière à ce que le temps moyen de séjour du lactame dans l'appareil de distillation soit inférieur ou égal à 1 heure.

5. Procédé selon l'une des revendications 1 à 4, **caractérisé en ce que** le lactame mis en oeuvre est choisi parmi ceux qui sont obtenus par hydrolyse cyclisante en phase vapeur, d'un aminonitrile aliphatique de formule générale (1) :
N≡C―R―NH₂ (1)
dans laquelle R représente un radical alkylène linéaire ou ramifié ayant de 3 à 12 atomes de carbone.

6. Procédé selon l'une des revendications 1 à 7, **caractérisé en ce que** le lactame mis en oeuvre est choisi parmi ceux qui sont obtenus par hydrolyse cyclisante en phase vapeur, d'un aminonitrile aliphatique de formule générale (1), dans laquelle R représente un radical alkylène linéaire ayant 3, 4, 5 ou 10 atomes de carbone.

7. Procédé selon l'une des revendications 1 à 8, **caractérisé en ce que** le lactame mis en oeuvre est le caprolactame.

8. Procédé selon la revendication 3, **caractérisé en ce que** la distillation est conduite de manière à ce que le temps moyen de séjour du lactame dans l'appareil de distillation soit inférieur ou égal à 30 minutes.

9. Procédé selon la revendication 6, **caractérisé en ce que** le lactame est stocké à une température inférieure ou égale à 40°C pendant une durée supérieure ou égale à 1 heure.

## Claims

1. Process for separating a lactam from the reaction flow obtained from a vapour-phase cyclizing hydrolysis of an aminonitrile, characteized in that the reaction flow leaving the hydrolysis reactor is cooled, over a period of less than or equal to 10 minutes:
- to a temperature of 50°C to 150°C before being distilled,
- or to a temperature below or equal to 50°C before being stored, for at least one hour, before being fractionated.

2. Process according to Claim 1, **characterized in that** the reaction flow is cooled to a temperature of from 70°C to 120°C before being distilled.

3. Process according to either of Claims 1 and 2, **characterized in that** the duration of feeding of the distillation column, that is to say the time which elapses between the reaction flow leaving the hydrolysis reactor and the actual distillation, is limited to a maximum of one hour and preferably to 30 minutes.

4. Process according to one of Claims 1 to 3, **characterized in that** the distillation is carried out such that the average residence time of the lactam in the distillation apparatus is less than or equal to 1 hour.

5. Process according to one of Claims 1 to 4, **characterized in that** the lactam used is chosen from those which are obtained by vapour-phase cyclizing hydrolysis of an aliphatic aminonitrile of general formula (1) :
N≡C-R-NH₂ (1)
in which R represents a linear or branched alkylene radical having from 3 to 12 carbon atoms.

6. Process according to one of Claims 1 to 5, **characterized in that** the lactam used is chosen from those which are obtained by vapour-phase cyclizing hydrolysis of an aliphatic aminonitrile of general formula (1), in which R represents a linear alkylene radical having 3, 4, 5 or 10 carbon atoms.

7. Process according to one of Claims 1 to 6, **characterized in that** the lactam used is capro-lactam.

8. Process according to Claim 3, **characterized in that** the distillation is carried out such that the average residence time of the lactam in the distillation apparatus is less than or equal to 30 minutes.

9. Process according to Claim 6, **characterized in that** the lactam is stored at a temperature below or equal to 40°C for a period of longer than-or equal to 1 hour.

## Patentansprüche

1. Verfahren zur Herstellung eines Lactams, ausgehend von einem Reaktionsstrom, der von einer cyclisierenden Hydrolyse eines Aminonitrils in der Dampfphase stammt, **dadurch gekennzeichnet, daß** der den Hydrolysereaktor verlassende Reaktionsstrom in einem Zeitraum von unter oder gleich 10 Minuten
- auf eine Temperatur von 50 °C bis 150 °C abgekühlt wird, bevor man ihn destilliert,
- auf eine Temperatur von unter oder gleich 50 °C abgekühlt wird, bevor man ihn während mindestens einer Stunde lagert und bevor man ihn fraktioniert.

2. Verfahren nach Anspruch 1, **dadurch gekennzeichnet, daß** der Reaktionsstrom auf eine Temperatur von 70 °C bis 120 °C abgekühlt wird, bevor man ihn destilliert.

3. Verfahren nach einem der Ansprüche 1 oder 2, **dadurch gekennzeichnet, daß** man die Dauer der Speisung der Destillationskolonne, das heißt, die Zeit, die zwischen dem Austritt des Reaktionsstromes aus dem Hydrolysereaktor und der eigentlichen Destillation abläuft, auf 1 Stunde maximal und vorzugsweise auf 30 Minuten begrenzt.

4. Verfahren nach einem der Ansprüche 1 bis 3, **dadurch gekennzeichnet, daß** die Destillation in der Weise durchgeführt wird, daß die mittlere Verweilzeit des Lactams in der Destillationsapparatur unter oder gleich 1 Stunde beträgt.

5. Verfahren nach einem der Ansprüche 1 bis 4, **dadurch gekennzeichnet, daß** das eingesetzte Lactam unter denjenigen ausgewählt wird, die durch cyclisierende Hydrolyse eines aliphatischen Aminonitrils der allgemeinen Formel (1)
N≡C-R-NH₂ (1)
in der Dampfphase erhalten werden, worin R einen geraden oder verzweigten Rest Alkylen mit 3 bis 12 Kohlenstoffatomen darstellt.

6. Verfahren nach einem der Ansprüche 1 bis 5, **dadurch gekennzeichnet, daß** das eingesetzte Lactam unter denjenigen ausgewählt wird, die durch cyclisierende Hydrolyse eines aliphatischen Aminonitrils der allgemeinen Formel (1) in der Dampfphase erhalten werden, worin R einen geraden Rest Alkylen mit 3, 4, 5 oder 10 Kohlenstoffatomen darstellt.

7. Verfahren nach einem der Ansprüche 1 bis 6, **dadurch gekennzeichnet, daß** das eingesetzte Lactam Caprolactam ist.

8. Verfahren nach Anspruch 3, **dadurch gekennzeichnet, daß** die Destillation in der Weise durchgeführt wird, daß die mittlere Verweilzeit des Lactams in der Destillationsapparatur unter oder gleich 30 Minuten beträgt.

9. Verfahren nach Anspruch 6, **dadurch gekennzeichnet, daß** das Lactam bei einer Temperatur von unter oder gleich 40 °C während einer Dauer von über oder gleich 1 Stunde gelagert wird.
